# EUROPÄISCHE PATENTSCHRIFT

(11) **EP 0 510 390 B1**
(45) Veröffentlichungstag und Bekanntmachung des Hinweises auf die Patenterteilung: **22.05.1996**
(21) Anmeldenummer: 92105529.9
(22) Anmeldetag: 31.03.1992
(51) Int. Cl.: A61B 17/58

(54) **Platte zum Abdecken eines Bohrloches in einem Schädeldach im Zusammenhang mit dem Fixieren eines Schädel-Knochendeckels**
Plate for covering a hole bored in the top of the skull in context with re-attaching a cover of bone to the skull
Plaque pour recouvrir un trou foré dans la calotte crânienne dans le contexte de la fixation d'une partie de cette calotte crânienne

(30) Priorität: 11.04.1991 DE 4111856
(43) Veröffentlichungstag der Anmeldung: 28.10.1992
(73) Patentinhaber: Leibinger GmbH, D-79111 Freiburg (DE)
(72) Erfinder: Leibinger, Karl, W-7200 Tuttlingen-Möhringen (DE); Leibinger, Franz, W-7202 Mühlheim-Stetten (DE)
(74) Vertreter: von Hellfeld, Axel, Dr. Dipl.-Phys.

(56) Entgegenhaltungen:
- EP-A- 0 290 138
- WO-A-88/01853
- DE-U- 9 014 613
- FR-A- 2 254 304
- FR-A- 2 631 539

## Beschreibung

Die Erfindung betrifft eine Platte zum Abdecken eines Bohrloches in einem Schädeldach im Zusammenhang mit dem Fixieren eines Schädel-Knochendeckels.
Eine derartige Platte ist aus der US-A-0 290 138 bekannt.

Bei neurochirurgischen, plastischen und craniofazialen Eingriffen am oder durch das menschliche Schädeldach werden nach Lösung der die Schädelkalotte abdeckenden Weichteile häufig großflächige Knochensegmente des Schädeldachs gelöst und am Ende des chirurgischen Eingriffs an derselben Stelle des Schädeldachs oder in verlagerter Stellung erneut befestigt (refixiert).

Bei der chirurgischen Behandlung craniofazialer Fehlbildungen werden häufig ein oder mehrere Knochensegmente des Schädeldachs entfernt und nach einer Modellierung entsprechend dem gewünschten kosmetischen Ergebnis in verlagerter Stellung wieder refixiert. Solche Eingriffe, die häufig bereits im Kleinkindalter durchgeführt werden, haben den Zweck, knöcherne Fehlbildungen des Schädeldachs zu korrigieren, um ein ungehindertes Wachstum des Gehirns zu ermöglichen und gleichzeitig auch das kosmetische Erscheinungsbild des Patienten zu verbessern.

Bei neurochirurgischen Eingriffen werden Knochendeckel des Gehirnschädels in verschiedenen Bereichen und Größen abgehoben, um einen Zugang zum Gehirn zu ermöglichen. In der Regel werden die so abgehobenen Knochensegmente (im Folgenden als Schädel-Knochendeckel bezeichnet) nach Beendigung des sogenannten Weichteileingriffes (also des Eingriffes in das Gehirn) in der ursprünglichen Position refixiert.

Die bei solchen Eingriffen angewandte Operationstechnik sieht häufig einen sogenannten Bügelschnitt (von Ohr zu Ohr über den höchsten Punkt des Schädeldachs) durch die Weichteile vor, wonach eine subperiostale Unterminierung erfolgt und die größten Weichteillappen nach vorne bzw. nach hinten abgeklappt werden.

Je nach Größe, Lage und geometrischer Form des abzuhebenden Knochendeckels werden mehrere Bohrungen durch die Schädeldecke angelegt. Handelt es sich bei dem abzuhebenden Knochendeckel beispielsweise um ein dreieckiges Schädeldachsegment, so werden in der Regel drei Bohrungen an den Eckpunkten des Knochendeckels durchgeführt. Anschließend werden mittels einer Säge (die zur Vermeidung von Duraverletzungen mit einer Führungsnase versehen ist) die sogenannten Verbindungsosteotomien zwischen den Bohrlöchern ausgeführt; bei einer dreieckigen Form des abzuhebenden Knochendeckels sind somit die Verbindungsosteotomien die Seiten eines sphärisch gekrümmten Dreiecks. Anschließend kann der Knochendeckel abgehoben werden, um den weiteren Eingriff durchzuführen.

Nach Beendigung des Eingriffs sind die zuvor abgehobenen Knochendeckel wieder zu refixieren, d.h. wieder an der gewünschten Stelle zu befestigen.

Für diese Refixation des Knochendeckels sind im Stand der Technik unterschiedliche Hilfsmittel bekannt. Die EP-A-0 290 138 und die EP-A-0 291 632 beschreiben für diesen Zweck sogenannte Kleinknochenplatten aus körperverträglichem Material, wie beispielsweise Titan oder einer Chromkobaltmolibdänlegierung, die streifenförmig gestaltet sind und in Abständen mit Löchern zur Aufnahme von Knochenschrauben versehen sind. Diese langgestreckten Platten sind so ausgelegt, daß sie vom Chirurgen verformt werden können, um sie dem Knochen im zu versorgenden Bereich anzupassen.

Die EP-A-0 347 658 beschreibt eine Knochenplatte für die Osteosynthese mit Löchern für die Aufnahme von Knochenschrauben und mit einem Zahnradgetriebe zwischen den Plattenteilen zur Verstellung der Relativlage der Plattenteile.

Auch die deutschen Gebrauchsmuster DE-U-85 28 003 und DE-U-87 06 912 beschreiben Knochenplatten, die zum Fixieren eines Schädel-Knochendeckels verwendet werden.

Die in der DE-C-40 28 021 veröffentlichte ältere Anmeldung beschreibt ein Osteosynthesegitter mit Löchern zur Aufnahme von Knochenschrauben (d.h. Befestigungsschrauben, die in den Knochen eingedreht werden).

Ein anderes Implantat sind die sogenannten Mesh-Systeme, insbesondere nach DUMBACH. Hier werden relativ großflächige, vom Chirurgen passgenau biegbare, gelöcherte Scheiben (z.B. aus Titan) als Implantat für die Knochenfixierung hauptsächlich im Kieferbereich verwendet.

Besondere Probleme werfen die oben erwähnten, zu Beginn einer Schädeleröffnung angelegten Bohrlöcher auf. Aus mehreren Gründen, unter anderem zum Schutz des postoperativ nur durch die Weichteile abgedeckten Gehirns und zur Verbesserung des kosmetischen Resultats, sollten die Bohrlöcher nach der Operation ebenfalls geschlossen sein. Beim Anlegen der Bohrlöcher unter Verwendung üblicher Bohrer bzw. Trephinen entsteht in aller

Regel allerdings kein Knochenpropfen, der refixiert werden könnte, sondern vielmehr fällt aufgrund der zerspanenden Wirkung des rotierenden Instrumentes nur Knochenmehl an. Das Problem der Bohrlöcher wird im Stand der Technik wie folgt zu lösen versucht:
- Die Bohrlöcher bleiben unverschlossen. Hierdurch ergibt sich jedoch nach der Heilungsphase ein zumeist unbefriedigendes kosmetisches Ergebnis, da, besonders im Bereich der Stirn, sichtbare "Dellen" entstehen, unter welchen oftmals sogar das Pulsieren des Gehirns sichtbar ist. Die Abdeckung des Bohrloches lediglich mittels der Weichteile bietet keinen ausreichenden Schutz des Gehirns vor Verletzungen.
- Das beim Anlegen der Bohrlöcher anfallende Knochenmehl kann teilweise aufgefangen und zur Abdeckung der Perforation verwendet werden. Bei diesem Verfahren ist jedoch keine ausreichende Stabilität des Knochens und auch kein vorhersehbares positives kosmetisches Ergebnis gewährleistet. Auch ist kein unmittelbarer Schutz des Gehirns gegeben.
- Es ist weiterhin bekannt, alloplastische Materialien, wie Knochenwachse, Knochenzement etc. zu verwenden. Auch diese Materialien bieten jedoch keinen sicheren Schutz des Gehirns, wobei hinzukommt, daß es sich in aller Regel um körperfremdes Implantatmaterial handelt, welches am Ort verbleibt und im Laufe der Zeit nicht umgebaut wird, so daß Infektionen oder andere unerwünschte Reaktionen des Körpers auftreten können.
- Schließlich ist es im Stand der Technik noch bekannt, Kunststoffabdeckungen zu verwenden, die über den Bohrlochrand ragen und im Bohrloch selbst verklemmt werden. Auch bei diesem Verfahren ist kein kosmetisch einwandfreies Ergebnis gewährleistet (die Kunststoffabdeckungen können sich über die Kontur des Knochens vorbilden). Aufgrund seiner Weichheit bietet der Kunststoff auch keinen sicheren Schutz gegen Stichverletzungen.

Der Erfindung liegt die Aufgabe zugrunde, bei den vorstehend erläuterten Problemen Abhilfe zu schaffen und eine Refixierung von Schädel-Knochendeckeln zu ermöglichen, die einen sicheren Schutz gegen Verletzungen gewährleistet, ohne den postoperativen Heilungsprozeß zu beeintrachtigen.

Gelöst wird diese Aufgabe bei einer Platte der eingangs genannten Art durch den kennzeichnenden Teil des Anspruchs 1

Die erfindungsgemäße Platte deckt das Bohrloch ab und ihre äußere Kontur ist bevorzugt im wesentlichen rund, insbesondere kreisförmig, oder polygonal. Die vorzugsweise leicht konkav geformte Abdeckplatte wird in verschiedenen Größen bereitgestellt und vom Chirurgen konzentrisch über dem Bohrloch angeordnet und dann mittels Knochenschrauben am Schädelknochen sicher verankert.

In der FR-A-2 631 539 ist eine ähnliche Lochplatte zur Verstärkung einer Gelenkpfanne beschrieben u. gezeigt.

Eine derartige Lochplatte muß sich über die gesamte Abmessung der Pfanne erstrecken u. ist nicht geeignet, die für den speziellen Zweck der Erfindung zugrundeliegende Aufgabe, insbesondere hinsichtlich des Heilungsprozesses, zu lösen.

Bevorzugt ist vorgesehen, daß die Löcher zur Aufnahme je einer Knochenschraube auf einem Kreis angeordnet sind, dessen Mittelpunkt mit dem Zentrum der Platte zusammenfällt.

Entlang dem Umfang der erfindungsgemäßen Platte sind die Löcher zur Aufnahme der Knochenschrauben bevorzugt in regelmäßigen Abständen angeordnet, wobei zumindest drei Löcher zur Schraubenaufnahme für eine fixationsstabile Drei-Punkt-Verankerung erforderlich und vorgesehen sind. Zusätzliche Löcher zur Schraubenaufnahme erweitern die Verankerungsmöglichkeiten der Platte.

Die Flügel der erfindungsgemäßen Platte sind im Inneren der Platte miteinander verbunden. Die gesamte Platte ist integral aus einem einzigen Material, wie Titan, Niob etc. hergestellt.

Die einzelnen Flügel der erfindungsgemäßen Platte sind durch sich radial erstreckende Schlitze voneinander getrennt. Die Schlitze erstrecken sich aber bevorzugt nur über einen Teil des Radius der Platte, um eine gute Stabilität bei der Refixierung zu erreichen. Bevorzugt entspricht die Schlitzlänge etwa dem halben Radius der Platte mit einer zugelassenen Abweichung von plus/minus 30 %, wobei der Radius bezogen ist auf den massiven Teil der Platte, also ohne eventuell vorgesehene zusätzliche Ringe am Äußeren der Platte zur Aufnahme von Löchern für die Schraubenaufnahme.

Gemäß einer bevorzugten Ausgestaltung sind die Schlitze zwischen den Flügeln der Platte konisch ausgeformt.

Die Schlitze haben unter anderem zwei Funktionen:
- Der Chirurg kann die Schlitze als "Sichtschlitze" ausnutzen und sieht durch sie hindurch den Rand des darunter angeordneten Bohrloches, wodurch die Platte in einfacher Weise mit konstanten Seitenabständen zum Bohrloch zentral verankert werden kann, d.h. die im wesentlichen runde oder rotationssymmetrische Platte kann konzentrisch zum Bohrloch fixiert werden.
- Bevorzugt werden die erfindungsgemäßen Platten in einer der Krümmung des Schädels angepaßten konkaven Form bereitgestellt. In Abhängigkeit von den Bedingungen des besonderen Einsatzes der Platte kann sie jedoch individuell geformt werden. Durch die Schlitze zwischen den einzelnen Flügeln der Platte lassen sich die Platten und insbesondere die Flügel selbst nahezu jeder anatomischen Situation anpassen, wodurch das erzielte kosmetische Ergebnis optimal wird.

Gemäß einer anderen Ausgestaltung der erfindungsgemäßen Platte ist in deren Zentrum eine zentrale Schraubenaufnahme vorgesehen für den Fall, daß ein eventuell erhaltener Knochenpropfen zusätzlich an der Plattenunterseite angeschraubt werden soll.

Die erfindungsgemäße Platte ist vorzugsweise so gestaltet, daß sie ein möglichst flaches Profil aufweist, wobei die zur Befestigung der Platte verwendeten Knochenschraubenköpfe möglichst ohne Überstehen in der Platte versenkt sind.

Die erfindungsgemäße Platte hat also eine Doppelfunktion: Zum einen dient sie als Bohrlochabdeckung und zum anderen dient sie der Fixation des Knochendeckels. Mindestens eine, vorzugsweise zwei Schrauben verbinden die Platte mit dem Knochendeckel, während die restlichen Schrauben eine Verbindung über das Bohrloch zum restlichen Schädeldach herstellen. Es kann eine große Anzahl verschieden dimensionierter Knochendeckel in allen Bereichen des Schädels durch Verwendung mindestens zweier, vorzugsweise dreier Platten am Schädeldach fixiert werden. Eine zusätzliche Stabilisierung der Schädelsegmente durch die eingangs genannten beschriebenen alternativen Fixationsmöglichkeiten gemäß dem Stand der Technik ist nicht erforderlich, steht aber im Bedarfsfall dem Chirurg ergänzend zur Verfügung.

Nachfolgend werden Ausführungsbeispiele der Erfindung anhand der Zeichnung näher beschrieben. Es zeigt:
- Fig. 1: schematisch einen craniofazialen Eingriff zur Behebung knöcherner Fehlbildungen, bei dem ein Schädel-Knochendeckel und mehrere Teilsegmente gelöst werden;
- Fig. 2 - 5: unterschiedliche Ausgestaltungen erfindungsgemäßer Platten zum Abdecken eines Bohrloches in einem Schädeldach und zum Fixieren eines Schädel-Knochendeckels; und
- Fig. 6: schematisch die Abdeckung von Bohrlöchern in einem Schädeldach und die Fixierung eines Knochendeckels am Schädeldach mittels erfindungsgemäßer Platten.

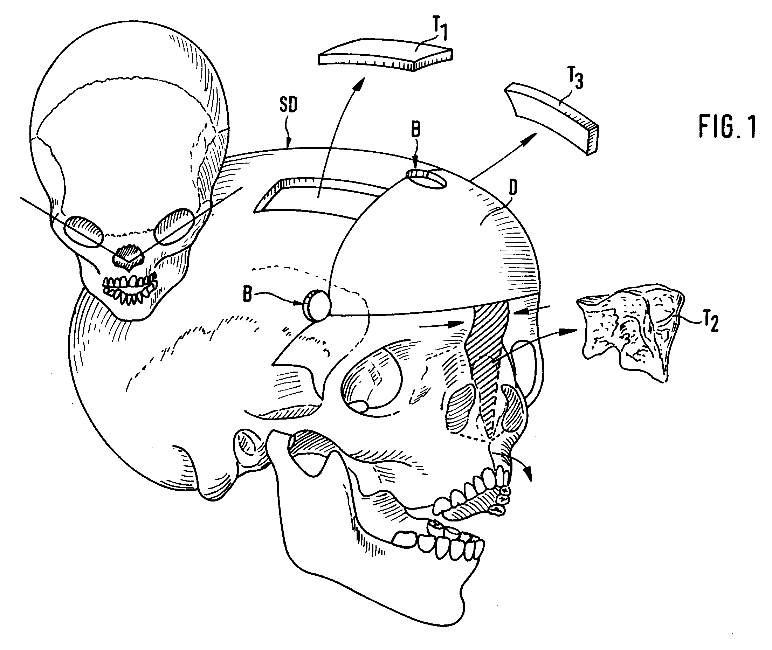

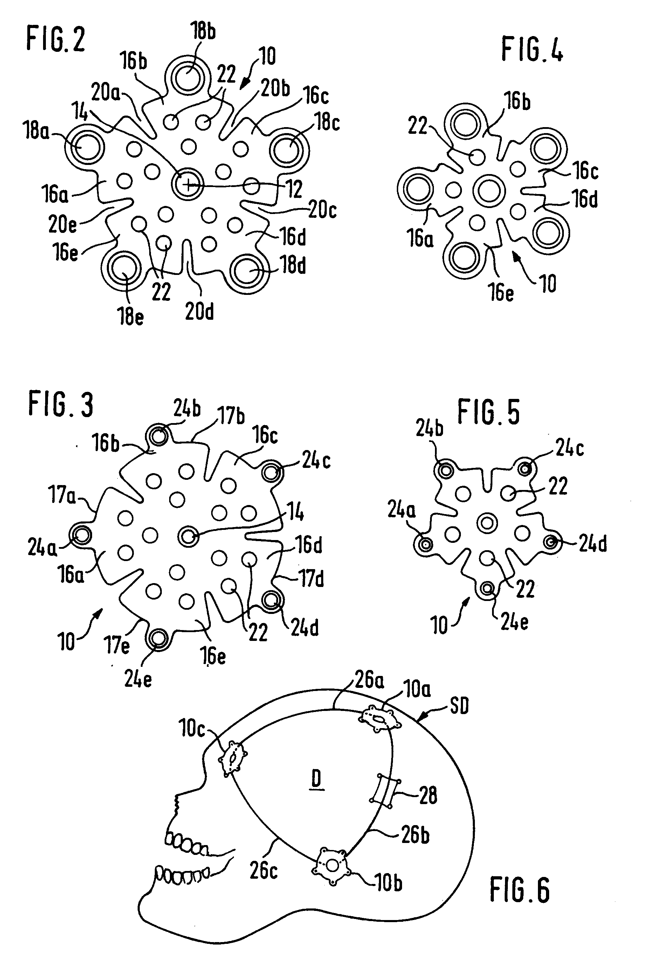

Fig.1 zeigt schematisch ein Schädeldach SD, in dem Bohrungen B angelegt sind. Zwischen den Bohrungen B verlaufen Schnittlinien (vgl. auch Fig.6), so daß ein Schädel-Knochendeckel D abnehmbar ist. Zur Behebung der in Fig.1 erkennbaren knöchernen Fehlbildung werden Teile T₁, T₂, T₃ vom Schädel entfernt.

Die vorliegende Erfindung betrifft die Refixierung des Schädel-Knochendeckels D und die Abdeckung der Bohrlöcher B.

Fig.2 zeigt ein erstes Ausführungsbeispiel einer Platte zum Abdecken eines Bohrloches B in einem Schädeldach SD und zum Fixieren eines Schädel-Knochendeckels D. Die Platte 10 dient als Implantat und ist integral aus einem biokompatiblen Werkstoff hergestellt, z.B. Titan oder Niob.

Fig.2 zeigt ebenso wie Fig.3 ein Ausführungsbeispiel zur Abdeckung eines relativ großen Bohrloches B mit Durchmessern bis zu 14 mm, während die Fig.4 und 5 Platten zeigen, die für kleinere Bohrlöcher mit Durchmessern bis zu 7 mm vorgesehen sind.

In den Fig.2 bis 5 sind einander entsprechende Bauteile mit gleichen Bezugszeichen versehen, so daß im einzelnen nur Fig.2 beschrieben zu werden braucht. Die Ausführungsbeispiele gemäß den Fig.3 bis 5 verstehen sich dann ohne weiteres.

Die Platte 10 ist rotationssymmetrisch in bezug auf ihr Zentrum 12 ausgebildet. Das Zentrum 12 befindet sich mittig in einem Loch 14.

Bei den dargestellten Ausführungsbeispielen weist die Platte 10 fünf Flügel 16a, 16b, 16c, 16d und 16e auf.

Zwischen den Flügeln 16a-16e sind jeweils Schlitze 20a, 20b, 20c, 20d und 20e angeordnet. Die Schlitze erstrecken sich radial in bezug auf das Zentrum 12 etwa über den halben Radius der Platte 10, wobei der Radius gemessen wird vom Zentrum 12 bis zu den Außenumfangsabschnitten 17a, 17b, 17c, 17d bzw. 17e der Flügel 16a-16e, d.h. der Radius schließt nicht ein die radialen Verlängerungen der einzelnen Flügel, in denen jeweils Löcher 18a, 18b, 18c, 18d und 18e zur Aufnahme von Knochenschrauben vorgesehen sind. Die Schlitze sind radial nach innen sich konisch verjüngend ausgebildet.

Die gesamte Platte 10 ist gleichmäßig mit Löchern 22 versehen, damit im Verlaufe und nach der Operation Blut und andere Flüssigkeit abfließen kann.

Die Löcher 18a-18e weisen jeweils Ansenkungen auf, damit die Köpfe von Knochenschrauben (nicht gezeigt) nicht vorstehen.

Die Platte 10 einschließlich der Flügel 16a-16e ist insgesamt konkav derart geformt, daß sie vorab grob an ein Schädeldach SD angepaßt ist. Aufgrund der beschriebenen Formgebung der Platte 10 und der Schlitze zwischen den Flügeln kann der Operateur vor Ort in Abhängigkeit von der gegebenen geometrischen Situation eine Anpassung der Form vornehmen und die Flügel und erforderlichenfalls auch das Innere der Platte wunschgemäß zurechtbiegen.

Die Löcher 18a-18e zur Aufnahme von Knochenschrauben sind in Ringen 24a-24e vorgesehen, die bei den gezeigten Ausführungsbeispielen außen als integrale Vorsprünge über die Außenumfangsabschnitte 17a-17e der Flügel 16a-16e vorstehen. Die Vorsprünge und die darin ausgeformten Löcher sind jeweils mittig am zugeordneten Flügel angeordnet.

Bei den in den Fig.2 bis 5 dargestellten Ausführungsbeispielen von erfindungsgemäßen Platten sind jeweils fünf Flügel vorgesehen. Dies hat sich als günstig erwiesen.

Fig.6 zeigt die Refixierung eines Schädel-Knochendeckels D an einem Schädeldach SD (vgl. auch Fig.1). Der Knochendeckel D hat die Form eines sphärischen Dreiecks, wobei die Bohrungen (in Fig.6 nicht zu erkennen, da durch die Platten abgedeckt) jeweils an den Eckpunkten des durch die Schnittlinien 26a, 26b und 26c gebildeten Dreiecks positioniert sind. Die erfindungsgemäßen Platten 10a, 10b und 10c sind konzentrisch über den Bohrlöchern überlappend angeordnet und fixieren den Deckel D in bezug auf das Schädeldach SD. Zusätzlich kann die Fixierung gefördert werden durch ein als solches bekanntes Gitter 28.

Die vorstehend beschriebenen Ausführungsbeispielen mit fünf Flügeln können dahingehend abgewandelt werden, daß insbesondere auch drei Flügel oder auch mehr als fünf Flügel vorgesehen sind. Bevorzugt werden eine ungerade Anzahl von Flügeln. Die bei den dargestellten Ausführungsbeispielen gezeigte Rotationssymmetrie (bei fünf Flügeln ein Rotationswinkel von 72°) erleichtert die Handhabung der Platte.

Die Platten gemäß den vorstehend beschriebenen Ausführungsbeispielen sind im wesentlichen (bis auf die angesetzten Ringe 24) rund, d.h. der Grundkörper ist im wesentlichen kreisförmig um das Zentrum 12. Es sind Abwandlungen gegenüber dieser bevorzugten Ausführungsform möglich, insbesondere quadratische oder rechteckige Grundkörper.

## Patentansprüche

1. Platte (10) zum Abdecken eines Bohrloches (B) in einem Schädeldach (SD) im Zusammenhang mit dem Fixieren eines Schädel-Knochendeckels (D), **gekennzeichnet** durch mehrere sich radial in bezug auf ein Zentrum (12) erstreckende Flügel (16a-16e), die durch Schlitze (20a-20e) voneinander getrennt sind und von denen zumindest ein Teil an seinen Außenumfangsabschnitten (17a-17e) jeweils ein Loch (18a-18e) zur Aufnahme je einer Knochenschraube aufweist u. durch im Innenabschitt u. in den Flügeln (16a-16e) der Platte (10) vorgesehene Bohrungen (22), damit postoperativ Blut u. Gewebeflüssigkeit ungehindert ausfließen können.

2. Platte nach Anspruch 1,
dadurch **gekennzeichnet,** daß die Löcher (18a-18e) zur Aufnahme je einer Knochenschraube auf einem Kreis angeordnet sind, dessen Mittelpunkt mit dem Zentrum (12) der Platte zusammenfällt.

3. Platte nach Anspruch 1 oder 2,
dadurch **gekennzeichnet,** daß die Löcher (18a-18e) zur Aufnahme je einer Knochenschraube jeweils mittig am zugeordneten Außenumfangsabschnitt (17a-17e) eines Flügels (16a-16e) angeordnet sind.

4. Platte nach einem der vorhergehenden Ansprüche,
dadurch **gekennzeichnet,** daß die Löcher (18a-18e) zur Aufnahme je einer Knochenschraube in Ringen (24a-24e) vorgesehen sind, die als integrale Vorsprünge über die Außenumfangsabschnitte (17a-17e) der Flügel (16a-16e) vorstehen.

5. Platte nach einem der vorhergehenden Ansprüche,
dadurch **gekennzeichnet,** daß die Platte (10) einschließlich der Flügel (16a-16e) konkav gekrümmt ist.

6. Platte nach Anspruch 5,
dadurch **gekennzeichnet,** daß die Schlitze (20a-20e) sich etwa über den halben Radius der Platte (10) erstrecken.

7. Platte nach einem der vorhergehenden Ansprüche,
dadurch **gekennzeichnet,** daß im Zentrum (12) der Platte ein zentrales Loch (14) vorgesehen ist.

8. Platte nach einem der vorhergehenden Ansprüche,
dadurch **gekennzeichnet,** daß die Flügel (16a-16e) rotationssymmetrisch in bezug auf das Zentrum (12) der Platte (10) ausgebildet sind, wobei insbesondere fünf Flügel vorgesehen sind.

9. Platte nach einem der vorhergehenden Ansprüche,
dadurch **gekennzeichnet,** daß die Schlitze (20a-20e) konisch sind.

## Claims

1. Plate (10) for covering a drill hole (B) in a skull cap (SD) in connection with the fixation of a cranial bone cover (D)
**characterized** by a plurality of vanes (16a-16e) which extend radially with respect to a centre (12) and which are separated from each other by slots (20a-20e), and of which at least some comprise at their outer peripheral portions (17a-17e) in each case a hole (18a-18e) for receiving a bone screw, and by bores (22) provided in the inner portion and in the vanes (16a-16e) of the plate (10) so that postoperatively blood and tissue fluid can flow out without hindrance.

2. Plate according to claim 1,
**characterized** in that the holes (18a-18e) for receiving a respecive bone screw are arranged on a circle of which the centre point coincides with the centre (12) of the plate.

3. Plate according to claim 1 or 2,
**characterized** in that the holes (18a-18e) for receiving a respective bone screw are arranged respectivelly centrally on the associated outer peripheral portion (17a-17e) of a vane (16a-16e).

4. Plate according to any one of the preceding claims,
**characterized** in that the holes (18a-18e) for receiving a respective bone screw are provided in rings (24a-24e) which extend as integral projections beyond the outer peripheral portions (17a-17e) of the vanes (16a-16e).

5. Plate according to any one of the preceding claims,
**characterized** in that the plate (10) including the vanes (16a-16e) is concavely curved.

6. Plate according to claim 5,
**characterized** in that the slots (10a-20e) extend substantially over half the radius of the plate (10).

7. Plate according to any one of the preceding claims,
**characterized** in that in the centre (12) of the plate a central hole (14) is provided.

8. Plate according to any one of the preceding claims,
**characterized** in that the vanes (16a-16e) are formed rotation-symmetrical with respect to the centre (12) of the plate (10), in particular five vanes being provided.

9. Plate according to any one of the preceding claims,
**characterized** in that the slots (20a-20e) are conical.

## Revendications

1. Plaque (10) destinée à couvrir un trou (B) dans une voûte crânienne (SD) dans le contexte de la fixation d'un couvercle osseux crânien (D), caractérisée par plusieurs ailes (16a à 16e) s'étendant radialement par rapport à un centre (12), séparées par des fentes (20a à 20e) et dont au moins une partie présente sur ses segments circonférentiels externes (17a à 17e) un trou (18a à 18e) destiné à loger une vis d'ostéosynthèse, et par des trous (22) prévus dans le segment interne et dans les ailes (16a à 16e) de la plaque (10) pour que le sang et la lymphe puissent s'écouler librement après l'intervention.

2. Plaque selon la revendication 1, caractérisée en ce que les trous (18a à 18e) destinés à loger chacun une vis d'ostéosynthèse sont disposés sur un cercle dont le centre coïncide avec le centre (12) de la plaque.

3. Plaque selon la revendication 1 ou 2, caractérisée en ce que les trous (18a à 18e) destinés à loger chacun une vis d'ostéosynthèse sont disposés au centre de chaque segment circonférentiel externe correspondant (17a à 17e) d'une aile (16a à 16e).

4. Plaque selon une des revendications précédentes, caractérisée en ce que les trous (18a à 18e) destinés à loger chacun une vis d'ostéosynthèse sont prévus dans des anneaux (24a à 24e) qui dépassent en tant que saillies monoblocs des segments circonférentiels externes (17a à 17e) des ailes (16a à 16e).

5. Plaque selon une des revendications précédentes, caractérisée en ce que la plaque (10), y compris les ailes (16a à 16e), a une courbure concave.

6. Plaque selon la revendication 5, caractérisée en ce que les fentes (20a à 20e) s'étendent approximativement sur la moitié du rayon de la plaque (10).

7. Plaque selon une des revendications précédentes, caractérisée en ce qu'un trou central (14) est prévu au centre (12) de la plaque.

8. Plaque selon une des revendications précédentes, caractérisée en ce que les ailes (16a à 16e) présentent une symétrie de rotation par rapport au centre (12) de la plaque (10), cinq ailes notamment étant prévues.

9. Plaque selon une des revendications précédentes, caractérisée en ce que les fentes (20a à 20e) sont coniques.
